(19) Europäisches Patentamt
European Patent Office
Office européen des brevets

(11) **EP 1 626 318 A1**

(12) **EUROPÄISCHE PATENTANMELDUNG**

(43) Veröffentlichungstag:
**15.02.2006 Patentblatt 2006/07**

(51) Int Cl.:
***G05B 19/042*** *(2006.01)* ***F16P 3/00*** *(2006.01)*

(21) Anmeldenummer: 05107177.7

(22) Anmeldetag: **04.08.2005**

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(30) Priorität: **04.08.2004 DE 102004037859**

(71) Anmelder: **SIEMENS AKTIENGESELLSCHAFT 80333 München (DE)**

(72) Erfinder:
• **Kagermeier, Robert**
  **90427 Nürnberg (DE)**
• **Medlar, Donal**
  **91085 Weisendorf (DE)**
• **Paschold, Johannes**
  **07407 Rudolstadt (DE)**
• **Sierk, Dietmar**
  **06120 Halle (DE)**

(54) **Vorrichtung und Verfahren zur Kollisionsvermeidung**

(57) Die vorliegende Erfindung betrifft ein medizinisches Gerät mit einer Sicherungseinrichtung mit mindestens einer Quelle (3) zum Aussenden eines optischen Signals (10), mindestens einem Sensor (2) zum Detektieren des von der Quelle (3) ausgesandten optischen Signals (10), wobei die Quelle (3) und der Sensor (2) so angeordnet sind, dass unerwünschte Objekte in einem zu überwachenden Bereich erkannt werden können, und einer Kontrolleinheit (9) zur Steuerung des Geräts (8), wobei die Kontrolleinheit (9) das Gerät (8) in einen sicheren Betriebszustand schaltet, wenn durch die Quelle (3) und den Sensor (2) ein unerwünschtes Objekt in dem zu überwachenden Bereiche erkannt wird.

Des weiteren betrifft die Erfindung ein Verfahren, um die Schritte mittels der erfindungsgemäßen Vorrichtung durchzuführen.

FIG 2

EP 1 626 318 A1

**Beschreibung**

Die vorliegende Erfindung betrifft ein medizinisches Gerät mit einer Sicherungseinrichtung gemäß Patentanspruch 1 und ein Sicherungsverfahren für ein medizinisches Gerät gemäß Patentanspruch 14.

Für medizinische Untersuchungen werden häufig Geräte eingesetzt, die durch ihre Funktionseigenschaft bedingt Bewegungen ausführen. Beispiel hierfür sind MRT, CT, Röntgen oder ähnliches. Aufgrund der Bewegungen der medizinischen Geräte besteht die Gefahr, dass während des Betriebs das Gerät mit Personen oder Gegenständen kollidiert, die sich im Gefahrenbereich befinden oder während des Betriebs versehentlich in den Gefahrenbereich gelangen. Darüber hinaus kann auch bei medizinischen Geräten, die keine Bewegungen ausführen, durch in Gefahrenbereichen befindliche Personen eine Gefahr für die Personen oder das Gerät selbst entstehen. Es besteht daher die Notwendigkeit einer entsprechenden Vorrichtung und eines Verfahrens, durch welches im Gefahrenbereich befindliche Personen oder Objekte erkannt und entsprechende Sicherheitsvorkehrungen getroffen werden können.

Eine Möglichkeit, den Aktionsradius einer bewegten Maschine oder den Bereich um ein Gerät herum zu überwachen, bietet die Anwendung von Laserscannern, welche eine winkelabhängige Entfernungsinformation liefern. Allerdings ist eine derartige Technologie äußerst kostenintensiv.

Eine weitere Möglichkeit besteht in der Anwendung einer Schaltleiste, die am Gerät selbst montiert wird und bei Kollision aufgrund der Druckveränderung auf die Kollision reagiert und dadurch einen sofortigen Stop der Bewegung des Geräts bewirkt.

Nachteilig bei dieser Methode ist jedoch die Nachlaufzeit der Maschine, das heißt die Zeitspanne vom Erkennen einer Kollision bis zum endgültigen Stop der Bewegung, sowie der benötigte Druck, welcher relativ hoch sein muss, und die Tatsache, dass durch die Verwendung einer Schaltleiste die Kollision erst erkannt wird, wenn sie schon geschehen ist.

Aufgabe der vorliegenden Erfindung ist es deshalb, eine Vorrichtung und ein Verfahren zu schaffen, das Gefahren rechtzeitig erkennt, leicht an die verschiedenen Betriebszustände der Anlage anzupassen und gleichzeitig kostengünstig ist.

Die Aufgabe wird erfindungsgemäß durch die in Patentanspruch 1 gekennzeichnete Vorrichtung und das in Patentanspruch 14 gekennzeichnete Verfahren gelöst. Die Erfindung wird in ihren Unteransprüchen weitergebildet.

Gemäß der vorliegenden Erfindung wird ein medizinisches Gerät mit einer Sicherungseinrichtung beschrieben mit mindestens einer Quelle zum Aussenden eines optischen Signals, mindestens einem Sensor zum Detektieren des von der Quelle ausgesandten optischen Signals, wobei die Quelle und der Sensor so angeordnet sind, dass unerwünschte Objekte in einem zu überwachenden Bereich erkannt werden können, und einer Kontrolleinheit zur Steuerung des Geräts, wobei die Kontrolleinheit das Gerät in einen sicheren Betriebszustand schaltet, wenn durch die Quelle und den Sensor ein unerwünschtes Objekt in dem zu überwachenden Bereiche erkannt wird.

Des weiteren wird gemäß der vorliegenden Erfindung ein Sicherungsverfahren für ein medizinisches Gerät beschrieben, welches die folgenden Schritte umfasst:

Aussenden eines optischen Signals mittels einer Quelle, detektieren des durch die Quelle ausgesandten optischen Signals durch einen Sensor, anordnen der Quelle und des Sensors in einer Weise, dass unerwünschte Objekte in einem zu überwachenden Bereich erkannt werden können, steuern des Geräts durch eine Kontrolleinheit und schalten des Geräts in einen sicheren Betriebszustand, wenn durch die Quelle und den Sensor ein unerwünschtes Objekt in dem zu überwachenden Bereich erkannt wird.

Durch die Verwendung einer Quelle-Sensor-Anordnung kann ein beliebiger Bereich um das Gerät herum überwacht werden, wodurch eine Gefahr rechtzeitig erkannt werden und der Betriebszustand des Geräts entsprechend angepasst werden kann. Des weiteren kann durch die Anordnung die Überwachung von Gefahrenbereichen an verschiedene Geräte und deren Bewegungsrichtungen adaptiert werden.

Weiter erlaubt die Verwendung handelsüblicher kostengünstiger Quellen und Sensoren die Möglichkeit, den Kostenaufwand gering zu halten.

Gemäß eines bevorzugten Ausführungsbeispiels der Erfindung kann die Kontrolleinheit auf Basis des von dem Sensor detektierten optischen Signals ermitteln, ob sich in Richtung des ausgesandten optischen Signals ein Objekt befindet, an welchem das optische Signal reflektiert wird.

Vorzugsweise berechnet die Kontrolleinheit auf Basis des von dem Sensor detektierten optischen Signals den Abstand zwischen der Quelle und dem Objekt, an welchem das optische Signal reflektiert wird.

Vorteilhafterweise ändert die Kontrolleinheit die Funktionsweise des Geräts oder stoppt das Gerät, wenn der ermittelte Abstand zu dem Objekt einen vorgegebenen Wert unterschreitet oder überschreitet.

Des weiteren senden vorteilhafterweise mindestens zwei Quellen zueinander parallel verlaufende optische Signale aus.

Vorzugsweise senden mindestens zwei Quellen zueinander nicht parallel verlaufende optische Signale aus.

Mindestens eine Quelle kann bewegbar sein, so dass die Richtung des ausgesandten optischen Signals variabel ist.

Vorteilhafterweise ist mindestens eine Quelle an dem Gerät angebracht.

Des weiteren ist vorteilhafterweise mindestens eine Quelle in unmittelbarer Nähe des Geräts angebracht.

Vorzugsweise ist jeder Quelle genau ein Sensor zugeordnet.

Des weiteren sind vorzugsweise die Quelle und der zugeordnete Sensor unmittelbar nebeneinander angeordnet.

Bei der Quelle kann es sich um eine Infrarot-Leuchtdiode handeln.

Des weiteren kann es sich bei dem Sensor um einen InfrarotSensor handeln.

Die Erfindung wird im Folgenden anhand von Figuren näher erläutert. Dabei zeigen

Fig. 1    ein schematisches Blockdiagramm der erfindungsgemäßen Vorrichtung,

Fig. 2    eine erste Ausführungsform der erfindungsgemäßen Vorrichtung,

Fig. 3    eine zweite Ausführungsform der erfindungsgemäßen Vorrichtung,

Fig. 4    eine dritte Ausführungsform der erfindungsgemäßen Vorrichtung, und

Fig. 5    eine schematische Darstellung des für die Entfernungsberechnung verwendeten Triangulationsprinzips.

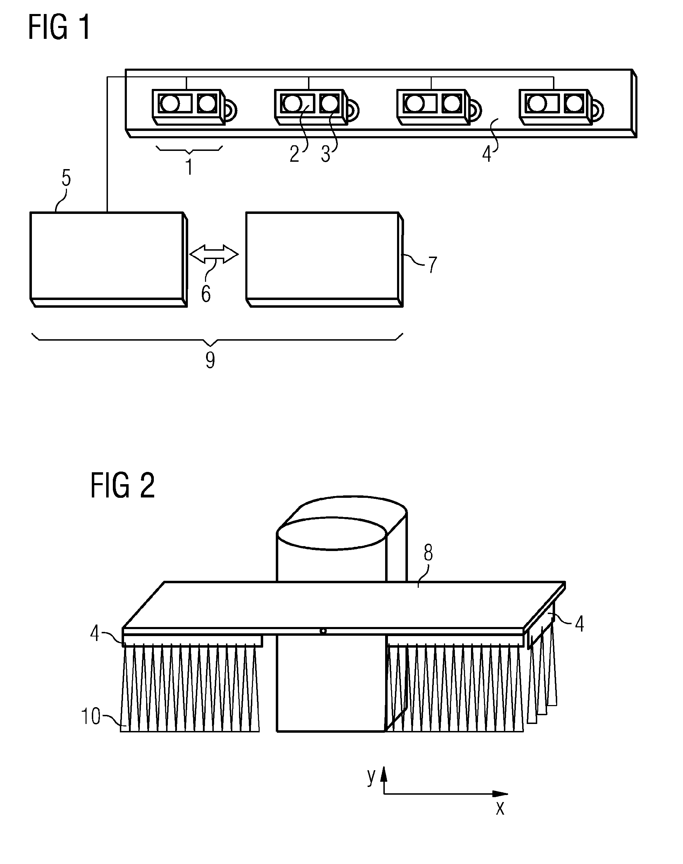

Fig. 1 zeigt ein schematisches Blockdiagramm der erfindungsgemäßen Vorrichtung. Hierbei sind jeweils eine Quelle 3 und ein Sensor 2 zu einer Anordnung 1 zusammengefasst, das heißt jeweils einer Quelle 3 ist ein Sensor 2 zugeordnet und die Quelle 3 sowie der zugeordnete Sensor 2 sind unmittelbar nebeneinander angeordnet. Die Quelle 3 dient zum Aussenden eines optischen Signals 10, vorzugsweise eines optischen Signals im Infrarotbereich. Als Quelle 3 kann hierfür eine Infrarot-Leuchtdiode verwendet werden. Der Sensor 2 dient zur Detektion des von der Quelle 3 ausgesandten optischen Signals 10, wobei es sich vorzugsweise bei dem Sensor 2 um einen Infrarotsensor handelt.

Für die Anordnung 1 aus Quelle 3 und Sensor 2 kann ein Gehäuse verwendet werden, in welchem die Quelle 3 und der Sensor 2 direkt nebeneinander in einem Abstand von ca. 1 cm angeordnet sind und in welchem notwendige optische Elemente, beispielsweise Linsen, zur Strahlbeeinflussung mit in das Gehäuse integriert sind. Vorzugsweise beträgt die Abmessung des Gehäuses ungefähr 4 cm in der Länge, 1 cm in der Breite und 1,5 cm in der Höhe. Jede Anordnung 1 ist hierbei fähig, den Abstand zu einem Objekt, welches sich vor der Anordnung 1 befindet, zu liefern.

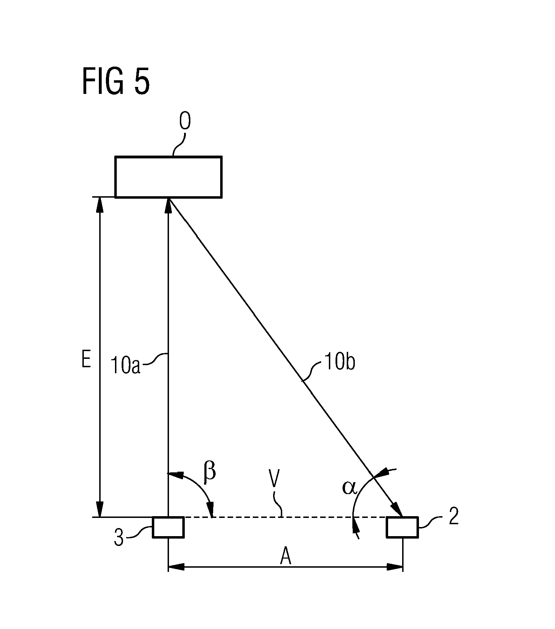

Das für die Entfernungsberechnung verwendete Triangulationsprinzip ist in Figur 5 schematisch dargestellt. Es sind die Quelle 3 und der Sensor 2 in einem Abstand A voneinander dargestellt. In einer zunächst unbekannten Entfernung E von der Quelle 3 befindet sich ein Objekt O. Die Quelle 3 sendet ein optisches Signal 10a aus, welches von dem Objekt O reflektiert und somit auf den Sensor 2 geleitet wird. Durch entsprechende Ansteuerung der Quelle 3 kann der Abstrahlwinkel β, welcher von der Verbindungslinie V zwischen der Quelle 3 und dem Sensor 2 und der Richtung des ausgesandten optischen Signals 10a eingeschlossen wird, beliebig verändert werden. Im vorliegenden Beispiel der Fig. 5 beträgt β genau 90°. Durch ein positionsempfindliches Detektorgerät sowie entsprechende Linsenanordnungen ist es dem Sensor 2 möglich, den Winkel α zu bestimmen, welcher von der Verbindungslinie V und dem von dem Objekt O reflektierten optischen Signal 10b eingeschlossen wird. Mit dem Winkel β=90°, einem bekannten Abstand A sowie einem bekannten Winkel α kann die Entfernung E zwischen der Quelle 3 und dem Objekt O bestimmt werden über die Winkelrelation in einem rechtwinkligen Dreieck, d.h. im vorliegenden Fall ergibt sich:

$$E = A \times \cot \alpha$$

In analoger Weise unter Verwendung entsprechender Dreieckssätze kann der Abstand A auch für Dreiecke, bei welchen β≠90° ist, berechnet werden.

Somit kann jede der in Fig. 1 dargestellten Anordnungen 1 aus Quelle 3 und Sensor 2 eine Entfernungsinformation liefern bezüglich eines vor der Quelle 3 befindlichen Objekts. Die maximale Reichweite einer solchen Anordnung 1 beträgt ungefähr 80 cm. Entfernungsinformation über dem maximalen Messbereich werden als unendlich gewertet. Es können mehrere Anordnungen 1 auf einer Schaltleiste 4 angebracht sein, so dass die Auswertung mehrerer Signale nebeneinander liegender Anordnungen 1 möglich wird, was den überwachten Bereich entsprechend vergrößert.

Eine Kontrolleinheit 9 überwacht hierbei die Ansteuerung, Erfassung und Auswertung der Messsignale. Die Kontrolleinheit 9 besteht aus einem Mikrokontroller 5, welcher für die direkte Ansteuerung der Quellen 3 und Sensoren 2 verantwortlich ist und die von den Sensoren gemessenen Werte erfasst. Der Mikrokontroller 5 kann die Sensoren 2 einzeln ansteuern, um so eine Beeinflussung durch benachbarte Sensoren 2 zu vermeiden.

Somit kann jede Entfernungsinformation einem einzelnen Sensor 2 zugeordnet werden. Des weiteren berechnet der Mikrokontroller 5 aus der Gesamtheit der von den Sensoren 2 gelieferten Messwerte, ob sich ein Objekt im Messbereich der Sensoren 2 befindet, wo das Objekt angeordnet ist, welche Größe es hat und ob es sich in irgendeine Richtung bewegt. Des weiteren kann der Mikrokontroller 5 den durch die Anordnungen 1 aus Quelle 3 und Sensor 2 überwachten Bereich an Bewegungen oder örtliche Gegebenheiten anpassen. Hierfür ist der Mikrokontroller 5 durch eine Schnittstelle 6, beispielsweise durch einen CAN-Bus, mit einer übergeordneten Steuerung 7 verbunden. Über die Schnittstelle 6 können die Detektionsergebnisse des Mikrokontrollers 5 an die übergeordnete Steuerung 7 weitergegeben werden, wodurch die übergeordnete Steuerung 7 die Ansteuerung der Quellen 3 und Sensoren 2 den Messergebnissen sowie äußeren Gegebenheiten anpassen kann. Beispielsweise kann eine selektive Aktivierung einzelner Sensoren 2 erfolgen oder es kann bestimmt werden, welcher Sensor zu welcher Zeit ein relevantes Signal liefern soll, bzw. welches Sensorsignal zur Auswertung herangezogen wird.

Durch die erfindungsgemäße Vorrichtung ist es möglich, durch die Anordnung mehrerer Sensoren eine Überwachungsfläche im dreidimensionalen Raum aufzuspannen. Eine Fläche kann von einem Bezugspunkt aus immer in zwei Richtungen aufgespannt werden. Legt man die Fläche beispielsweise in ein Koordinatensystem, so lässt sich die Fläche aus dem Ursprung in X- und Y-Richtung aufspannen, wie in den Fig. 2 bis 4 dargestellt. Das Aufspannen und damit auch Begrenzen in X-Richtung erfolgt durch die Anordnung von mehreren Quellen 3 und Sensoren 2 nebeneinander. Jede Anordnung 1 erweitert den Überwachungsbereich in X-Richtung. Die Gesamtheit der nebeneinander platzierten Anordnungen 1 bestimmen die Breite der aufgespannten Fläche. Die Begrenzung der Fläche in Y-Richtung erfolgt durch die Entfernungsinformation des Sensors 2. Somit wird die Fläche in Y-Richtung entweder durch den maximalen Sensorbereich begrenzt, wodurch dann auch keine Reflexion des optischen Signals 10 durch den Sensor 2 detektiert würde, oder die Fläche wird durch Objekte wie beispielsweise Wände oder Boden begrenzt. In diesem Falle würde das optische Signal 10 durch die Wände oder den Boden reflektiert und durch den Sensor 2 detektiert werden.

Durch dieses Prinzip ist es nicht notwendig, eine Quelle 3 und einen Sensor 2 gegenüber anzuordnen, wie es beispielsweise bei Lichtschranken der Fall ist, wodurch eine große Flexibilität für die aufgespannte Fläche erreicht wird und es möglich ist, die Fläche in beliebige Richtungen des Raumes aufzuspannen.

In Fig. 2 ist ein erstes Ausführungsbeispiel der erfindungsgemäßen Vorrichtung dargestellt. Hierbei sind an einem Gerät 8 für eine medizinische Untersuchung, beispielsweise MRT, CT, Röntgen oder ähnliches, Schaltleisten 4 mit mehreren nebeneinander platzierten Anordnungen 1 aus Quelle 3 und Sensor 2 angebracht, so dass durch die Anordnung entlang der Schaltleiste 4 sowie die ausgesendeten optischen Signale 10 eine rechtwinklige Überwachungsfläche aufgespannt wird.

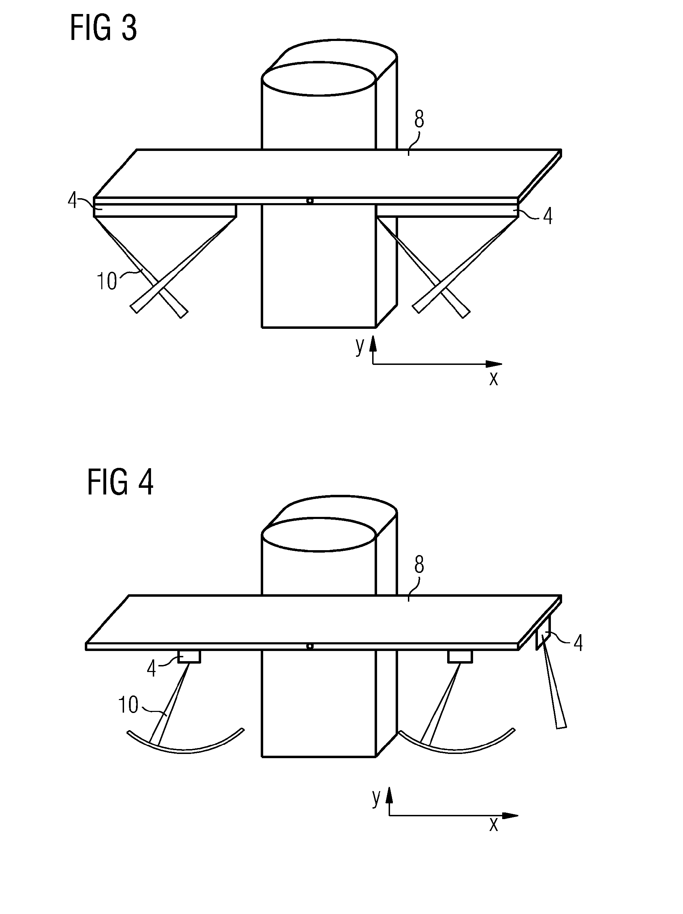

Fig. 3 zeigt ein zweites Ausführungsbeispiel der erfindungsgemäßen Vorrichtung. Hierbei sind, um die Zahl der verwendeten Anordnungen 1 zu verringern, jeweils nur zwei Anordnungen 1 auf einer Schaltleiste 4 angebracht, und die ausgesandten optischen Signale 10 sind nicht zueinander parallel sondern überkreuzt angeordnet.

Fig. 4 zeigt ein drittes Ausführungsbeispiel der erfindungsgemäßen Vorrichtung, bei welchen jeweils nur eine Anordnung 1 an der Schaltleiste 4 angebracht ist, wobei die Quelle 3 beweglich ist, so dass die Richtung des ausgesandten optischen Signals 10 variabel ist. Die hierdurch aufgespannte Fläche wird in X-Richtung durch die bekannte Winkelauslegung der Quelle 3 definiert und in Y-Richtung durch die maximal erfassbare Distanz. Die Fläche hat somit die Form eines Kreisbogenausschnitts.

Es sei angemerkt, dass die Erfindung nicht auf die eben beschriebene Ausführungsformen beschränkt ist, vielmehr sind Kombinationen der verschiedenen Ausführungsformen miteinander möglich, des weiteren können die Schaltleisten 4 entweder am Gerät 8 selbst oder in der Nähe des Geräts 8 angebracht sein.

Die Kontrolleinheit kann aufgrund der Sensorsignale, der bekannten Bewegungsrichtung des Geräts 8 sowie der bekannten räumlichen Umgebung aus den Signalen berechnen, ob es sich um ungefährliche Objekte im Bereich der Sensoren 2 handelt, beispielsweise Boden oder Wand, oder ob es sich um im Gefahrenbereich befindliche Personen oder Objekte handelt. Im Falle, dass eine Kollision des Geräts 8 mit im Gefahrenbereich befindlichen Objekten oder Personen droht, veranlasst die Kontrolleinheit einen Stop des Ablaufs des Geräts 8 oder eine Änderung der Funktionsweise des Geräts 8, um eine Kollision zu verhindern und somit Schaden für Personen oder Geräte vorzubeugen.

Da die Schaltleisten 4 sowohl am Gerät 8 selbst als auch in der Nähe des Geräts 8 angebracht werden können, ist es möglich, eine Überwachungsfläche in beliebigen Abstand sowie in beliebiger Position bezüglich des Geräts 8 aufzuspannen, und somit den Überwachungsbereich anzupassen.

Durch die Verwendung von Infrarot-Leuchtdioden und Infrarotsensoren und somit von Licht im nicht sichtbaren Bereich, wird eine störende Wahrnehmung durch in der Nähe des Geräts 8 befindliche Personen verhindert. Des weiteren kann die verwendete Lichtenergie so gering gehalten werden, dass das System auch im Nahbereich für die Augen unschädlich ist. Dies stellt einen weiteren Vorteil gegenüber laserbasierten Systemen dar.

**Patentansprüche**

1. Medizinisches Gerät mit einer Sicherungseinrichtung mit mindestens einer Quelle (3) zum Aussenden eines optischen Signals (10),
   mindestens einem Sensor (2) zum Detektieren des von der Quelle (3) ausgesandten optischen Signals (10),
   wobei die Quelle (3) und der Sensor (2) so angeordnet sind, dass unerwünschte Objekte in einem zu überwachenden Bereich erkannt werden können, und

einer Kontrolleinheit (9) zur Steuerung des Geräts (8), wobei die Kontrolleinheit (9) das Gerät (8) in einen sicheren Betriebszustand schaltet, wenn durch die Quelle (3) und den Sensor (2) ein unerwünschtes Objekt in dem zu überwachenden Bereiche erkannt wird.

2. Gerät nach Anspruch 1,
**dadurch gekennzeichnet, dass** die Kontrolleinheit (9) auf Basis des von dem Sensor (2) detektierten optischen Signals (10) ermitteln kann, ob sich in Richtung des ausgesandten optischen Signals (10) ein Objekt befindet, an welchem das optische Signal reflektiert wird.

3. Gerät nach Anspruch 2,
**dadurch gekennzeichnet,**
**dass** die Kontrolleinheit (9) auf Basis des von dem Sensor (2) detektierten optischen Signals (10) den Abstand zwischen der Quelle (3) und dem Objekt, an welchem das optische Signal reflektiert wird, berechnet.

4. Gerät nach Anspruch 3,
**dadurch gekennzeichnet, dass** die Kontrolleinheit (9) die Funktionsweise des Geräts (8) ändert oder das Gerät (8) stoppt, wenn der ermittelte Abstand zu dem Objekt einen vorgegebenen Wert unterschreitet oder überschreitet.

5. Gerät nach einem der Ansprüche 1 bis 4,
**dadurch gekennzeichnet, dass** mindestens zwei Quellen (3) zueinander parallel verlaufende optische Signale (10) aussenden.

6. Gerät nach einem der Ansprüche 1 bis 5,
**dadurch gekennzeichnet, dass** mindestens zwei Quellen (3) zueinander nicht parallel verlaufende optische Signale (10) aussenden.

7. Gerät nach einem der Ansprüche 1 bis 6,
**dadurch gekennzeichnet, dass** mindestens eine Quelle (3) bewegbar ist, so dass die Richtung des ausgesandten optischen Signals (10) variabel ist.

8. Gerät nach einem der Ansprüche 1 bis 7,
**dadurch gekennzeichnet, dass** mindestens eine Quelle (3) an dem Gerät (8) angebracht ist.

9. Gerät nach einem der Ansprüche 1 bis 8,
**dadurch gekennzeichnet, dass** mindestens eine Quelle (3) in unmittelbarer Nähe des Geräts (8) angebracht ist.

10. Gerät nach einem der Ansprüche 1 bis 9,
**dadurch gekennzeichnet, dass** jeder Quelle (3) genau ein Sensor zugeordnet ist.

11. Gerät nach Anspruch 10,
**dadurch gekennzeichnet, dass** die Quelle (3) und der zugeordnete Sensor (2) unmittelbar nebeneinander angeordnet sind.

12. Gerät nach einem der Ansprüche 1 bis 11,
**dadurch gekennzeichnet,**
**dass** es sich bei der Quelle (3) um eine IR-Leuchtdiode handelt.

13. Gerät nach einem der Ansprüche 1 bis 12,
**dadurch gekennzeichnet, dass** es sich bei dem Sensor (2) um einen IR-Sensor handelt.

14. Sicherungsverfahren für ein medizinisches Gerät umfassend die Schritte:

Aussenden eines optischen Signals (10) mittels einer Quelle (3),
Detektieren des durch die Quelle (3) ausgesandten optischen Signals (10) durch einen Sensor (2),
Anordnen der Quelle (3) und des Sensors (2) in einer Weise, dass unerwünschte Objekte in einem zu überwachenden Bereich erkannt werden können,
Steuern des Geräts (8) durch eine Kontrolleinheit (9) und schalten des Geräts (8) in einen sicheren Betriebszustand, wenn durch die Quelle (3) und den Sensor (2) ein unerwünschtes Objekt in dem zu überwachenden Bereich erkannt wird.

15. Verfahren nach Anspruch 14,
**gekennzeichnet durch** Ermitteln auf Basis des von dem Sensor (2) detektierten optischen Signals (10), ob sich in Richtung des ausgesandten optischen Signals (10) ein Objekt befindet, an welchem das optische Signal reflektiert wird.

16. Verfahren nach Anspruch 15,
**gekennzeichnet durch** Berechnen des Abstands zwischen der Quelle (3) und dem Objekt, an welchem das optische Signal (10) reflektiert wird, auf Basis des von dem Sensor (2) detektierten optischen Signals (10).

17. Verfahren nach Anspruch 16,
**gekennzeichnet durch** Ändern der Funktionsweise des Geräts (8) oder Stoppen des Geräts (8), wenn der ermittelte Abstand zu dem Objekt einen vorgegebenen Wert unterschreitet oder überschreitet.

18. Verfahren nach einem der Ansprüche 14 bis 17,
**gekennzeichnet durch** Aussenden mindestens zweier zueinander parallel verlaufender optischer Signale (10).

**19.** Verfahren nach einem der Ansprüche 14 bis 18, **gekennzeichnet durch** Aussenden mindestens zweier zueinander nicht parallel verlaufender optischer Signale (10).

**20.** Verfahren nach einem der Ansprüche 14 bis 19, **gekennzeichnet durch** Vorsehen mindestens einer bewegbaren Quelle (3), so dass die Richtung des ausgesandten optischen Signals (10) variabel ist.

**21.** Verfahren nach einem der Ansprüche 12 bis 20, **gekennzeichnet durch** Anbringen mindestens einer Quelle (3) an dem Gerät (8).

**22.** Verfahren nach einem der Ansprüche 14 bis 21, **gekennzeichnet durch** Anbringen mindestens einer Quelle (3) in unmittelbarer Nähe des Geräts (8).

**23.** Verfahren nach einem der Ansprüche 14 bis 22, **gekennzeichnet durch** Zuordnen genau eines Sensors (2) zu jeder Quelle (3).

**24.** Verfahren nach Anspruch 23, **gekennzeichnet durch** Anordnen der Quelle (3) und des zugeordneten Sensors (2) unmittelbar nebeneinander.

FIG 1

FIG 2

FIG 3

FIG 4

FIG 5

**Europäisches Patentamt**

# EUROPÄISCHER RECHERCHENBERICHT

Nummer der Anmeldung

EP 05 10 7177

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 102 16 122 A1 (SICK AG) 30. Oktober 2003 (2003-10-30) * Absätze [0001] - [0005] * * Absätze [0009], [0013], [0016] * * Abbildung 1 * ----- | 1-24 | G05B19/042 F16P3/00 |
| P,X | DE 103 14 852 A1 (SICK AG) 14. Oktober 2004 (2004-10-14) * Absätze [0001] - [0015] * * Abbildungen 1,2 * ----- | 1-24 | |
| A | DE 296 23 747 U1 (SIEMENS AG) 8. Juli 1999 (1999-07-08) * das ganze Dokument * ----- | 1-24 | |
| A | DE 41 26 168 A1 (SIEMENS AG, 8000 MUENCHEN, DE) 27. Februar 1992 (1992-02-27) * das ganze Dokument * ----- | 1-24 | |
| A | DE 31 23 893 A1 (SIEMENS AG) 5. Januar 1983 (1983-01-05) * das ganze Dokument * ----- | 1-24 | RECHERCHIERTE SACHGEBIETE (IPC) G05B F16P |
| A | US 2004/088079 A1 (LAVAREC ERWAN ET AL) 6. Mai 2004 (2004-05-06) * das ganze Dokument * ----- | 1-3, 14-16 | |
| A | DE 35 09 096 A1 (SAELZER,HEINRICH) 25. September 1986 (1986-09-25) * Seite 4, Zeile 1 - Seite 5, Zeile 2 * * das ganze Dokument * ----- | 1-24 | |

Der vorliegende Recherchenbericht wurde für alle Patentansprüche erstellt

| Recherchenort | Abschlußdatum der Recherche | Prüfer |
|---|---|---|
| München | 22. Dezember 2005 | Pöllmann, H.M. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**          EP 05 10 7177

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

22-12-2005

| Im Recherchenbericht angeführtes Patentdokument | Datum der Veröffentlichung | Mitglied(er) der Patentfamilie | Datum der Veröffentlichung |
|---|---|---|---|
| DE 10216122 A1 | 30-10-2003 | AT 280957 T<br>EP 1353196 A1<br>JP 2004005542 A<br>US 2003218122 A1 | 15-11-2004<br>15-10-2003<br>08-01-2004<br>27-11-2003 |
| DE 10314852 A1 | 14-10-2004 | AT 307344 T<br>EP 1467228 A2 | 15-11-2005<br>13-10-2004 |
| DE 29623747 U1 | 08-07-1999 | KEINE | |
| DE 4126168 A1 | 27-02-1992 | KEINE | |
| DE 3123893 A1 | 05-01-1983 | KEINE | |
| US 2004088079 A1 | 06-05-2004 | AT 298094 T<br>BR 0206753 A<br>CA 2435325 A1<br>CN 1489702 A<br>DE 60204659 D1<br>EP 1354220 A1<br>FR 2820216 A1<br>WO 02059646 A1<br>JP 2004522147 T | 15-07-2005<br>10-02-2004<br>01-08-2002<br>14-04-2004<br>21-07-2005<br>22-10-2003<br>02-08-2002<br>01-08-2002<br>22-07-2004 |
| DE 3509096 A1 | 25-09-1986 | KEINE | |

EPO FORM P0461

Für nähere Einzelheiten zu diesem Anhang : siehe Amtsblatt des Europäischen Patentamts, Nr.12/82